(19) Europäisches Patentamt
European Patent Office
Office européen des brevets

(11) Veröffentlichungsnummer: **0 154 907**
**A2**

(12) # EUROPÄISCHE PATENTANMELDUNG

(21) Anmeldenummer: **85102292.1**

(51) Int. Cl.⁴: **A 61 L 15/03**

(22) Anmeldetag: **01.03.85**

(30) Priorität: **13.03.84 DE 3409079**

(43) Veröffentlichungstag der Anmeldung: **18.09.85**
**Patentblatt 85/38**

(84) Benannte Vertragsstaaten: **AT BE CH DE FR GB IT LI NL SE**

(71) Anmelder: **BAYER AG, Konzernverwaltung RP**
**Patentabteilung, D-5090 Leverkusen 1 Bayerwerk (DE)**

(72) Erfinder: **von Bittera, Miklos, Max-Scheler Strasse 7,**
**D-5090 Leverkusen 3 (DE)**

(54) **Medizinische Pflaster.**

(57) Die Erfindung bezieht sich auf medizinische Pflaster zur Abgabe eines Wirkstoffes an die Haut über einen längeren Zeitraum, insbesondere auf antiphlogistische – medizinische Pflaster, deren Deckschicht aus einem längs-querelastischen, mit einem Polymer imprägnierten oder beschichteten textilen Flächengebilde, insbesondere Gewirk oder Gestrick besteht.

EP 0 154 907 A2

0154907

BAYER AKTIENGESELLSCHAFT
Konzernverwaltung RP
Patentabteilung

5090 Leverkusen, Bayerwerk

Ad/am

## Medizinische Pflaster

Die Erfindung bezieht sich auf medizinische Pflaster zur Abgabe eines Wirkstoffes an die Haut über einen längeren Zeitraum, insbesondere auf antiphlogistische - medizinische Pflaster, deren Deckschicht aus einem längs-querelastischen, mit einem Polymer imprägnierten oder beschichteten textilen Flächengebilde, insbesondere Gewirk oder Gestrick besteht.

In der US-PS 4 031 894 werden medizinische Pflaster beschrieben, die ein Reservoir aus Mineralöl und Polyisobuten besitzen. Das Polyisobuten stellt eine Mischung aus Komponenten verschiedener Molgewichte dar, nämlich PIB von M.G. 35.000 - 50.000 und 1.000.000 - 1.500.000.

Dieses Pflaster ist nur für Wirkstoffe, die in sehr geringen Dosen appliziert werden müssen, geeignet. In der US-PS wird Scopolamin genannt.

Es ist daher eine Aufgabe der vorliegenden Erfindung, medizinische Pflaster zu entwickeln, mit deren Hilfe über einen längeren Zeitraum geregelte, größere, therapeutisch wirksame Mengen eines Wirkstoffs über die Haut verabreicht werden können. Diese Pflaster sollen besonders geeignet sein zur Verabreichung von Antiphlogistika. Sie sollen mit der Haut verträglich sein und mit ihrer Hilfe soll es möglich sein, hohe therapeutisch wirksame Dosen des Wirkstoffs zu verabreichen.

Bekannte Wirkstoffabgabesysteme, wie z. B. Gele, Salben, Pflaster u. ä. erlauben nur eine begrenzte Wirkstoffresorption durch die Haut. Die Resorption hängt von der Grundlage und den Wirkstoffeigenschaften ab.

Gegenstand der Erfindung sind medizinische Pflaster zur Verabreichung eines Wirkstoffs an die Haut, enthaltend eine Deckschicht, die aus einem längs-querelastischen, mit einem Polymer imprägnierten oder beschichteten textil Flächengebilde, insbesondere Gewirk oder Gestrick besteht eine Reservoirschicht und eine abziehbare Schutzschicht, wobei die Reservoirschicht ein Polymer bestehend aus Poly isobutylen und/oder dessen Copolymerisate, ein Schleppmittel und ein Harz enthält.
Unter Polymer im Sinne der vorliegenden Erfindung werden Polyisobutylen und/oder dessen Copolymerisate verstanden.

Als Polyisobutylene im Sinne der Erfindung werden Polyisobutylene verstanden, die herstellungsbedingt eine Molmassen-Verteilung $M_w/M_n$ von 1,5 bis 3,5, vorzugsweise 2,0 bis 3,0, und ein Viskositätsmittel der Molmasse - wiederum herstellungsbedingt - von 30.000 bis zu 4.000.000 g/mol aufweisen. Vorzugsweise beträgt das Viskositätsmittel der erfindungsgemäß einzusetzenden Polyisobutylene 50.000 bis 1.000.000 g/mol, besonders bevorzugt 80.000 bis 500.000 g/mol. Die Viskositätsmittel lassen sich in bekannter Weise bestimmen gemäß Polymer-Handbook, J. Brandrup und F.H. Immergut, Wiley & Sons, New York, 1975, Kap. IV, S. 35.

Diese Polyisobutylene sind seit langem bekannt und können z.B. gemäß US-PS 2 203 873 oder gemäß DR-P 704 038 mit sauren Katalysatoren hergestellt werden.

Copolymerisate des Isobutylens im Sinne der Erfindung sind die des Isobutylens mit 0,5 - 5 Mol-% konjugierten Diolefinen, vorzugsweise solchen mit 4-6 C-Atomen, wie z.B. Butadien-1,3, Piperylen, 2,3-Dimethylbutadien, besonders bevorzugt mit Isopren, deren Molmassen von 30.000 bis 200.000 g/mol betragen können. Auch diese Isobuten-Copolymere sind bekannt. Ganz besonders bevorzugt werden Polyisobutylen-Homopolymerisate mit einem Viskositätsmittel von 80.000 bis 500.000 eingesetzt.

Unter Schleppmittel im Sinne der vorliegenden Erfindung werden Öle, Fettsäureester, Triglyceride, Alkohole und/ oder Fettsäuren verstanden.

Unter Ölen im Sinne der vorliegenden Erfindung werden hochsiedende, aliphatische, araliphatische und/oder aromatische Kohlenwasserstoffe verstanden, vorzugsweise Paraffinöl, Purcellinöl, Perhydrosqualen- und Lösungen von mikrokristallinen Wachsen in den Ölen, Mineralöle, bevorzugt Öle, deren Siedebereich zwischen 150°C und 400°C liegt; ferner ungesättigte Kohlenwasserstoffe mit mindestens 16 C-Atomen wie z.B. Oligomere von Monoolefinen wie Tetraisobutylen, Pentaisobutylen, Hexaisobutylen oder auch flüssige Polymerisate aus Dien(Monoen)-(Co)-Polymerisaten. Beispiele für flüssige Polymerisate aus konjugierten Dienen sind solche aus Butadien, Isopren, 1,3-Pentadien, 2,3-Dimethylbutadien, Copolymerisate verschiedener Diene sowie auch flüssige Copolymerisate aus einem konjugierten Diolefin und geringen Mengen von Monoolefinen wie z.B. Buten-1, Isobuten, Hexen-1, Octen-1, Styrol mit MG von 400 bis 6000, vorzugsweise 800 bis 3000 sowie Iodzahlen von 200 bis 500 und Viskositäten von 100 - 10.000 cP bei 50°C.

Besonders bevorzugt sind flüssige Polybutadien-Polymerisate, die zu mindestens 90 % 1,4-verknüpft sind, deren Anteil an cis-Doppelbindungen mehr als 60 % beträgt und deren Molmassen 1000 - 4000 betragen.

Unter Ölen werden auch Silikonöle verschiedener Viskosität, vorzugsweise mit mittleren Molgewichten von 312 bis 15.000, besonders bevorzugt Polydimethylsiloxane, verstanden.

Unter Fettsäureestern werden solche verstanden, die mindestens 12 C-Atome, vorzugsweise 15 bis 46 C-Atome, besonders bevorzugt 16 bis 36 C-Atome enthalten. Insbesondere werden darunter verstanden: Ethylstearat, Laurinsäurehexylester, Dipropylen-glykolpelargonat, Palmitinsäurecetylester, Isopropylmyristat, Isopropylpalmitat, Capryl/Caprinsäureester von gesättigten Fettalkoholen der Kettenlänge $C_{12}$ - $C_{18}$, Isopropylstearat, Ölsäureoleylester, Ölsäuredecylester, Ethyloleat, künstliches Entenbürzeldrüsenfett, und zwar jeweils einzeln oder im Gemisch.

Unter Triglyceriden werden reine oder gemischte Ester des Glycerins mit Fettsäuren der Kettenlänge $C_8$ - $C_{18}$ verstanden, vorzugsweise Capryl- und/oder Caprinsäuretriglyceride.

Unter Fettsäuren werden gesättigte oder ungesättigte Fettsäuren, vorzugsweise solche mit 12 - 24 C-Atomen, einzeln oder im Gemisch miteinander, besonders bevorzugt Ölsäure, verstanden.

Unter Ölen im Sinne der Erfindung werden ferner verstanden:
Süßmandelöl, Avocadoöl, Sesamöl,Rizinusöl, Olivenöl, Traubenkernöl, Nelkenöl, Erdnußöl, Maisöl, Haselnußöl, Jojobaöl, Carthamaöl und Weizenkeimöl, jeweils einzeln oder im Gemisch.

Unter Harzen im Sinne der vorliegenden Erfindung werden Kolophonium, dehydriertes Kolophonium, Glycerinester von dehydriertem Kolophonium, Glycerinester von Kolophoniumgummi, hydriertes Kolophonium, Glycerinester von hydriertem Kolophonium, Pentaerythritester von hydriertem Kolophonium, Methylester von hydriertem Kolophonium, polymerisiertes Kolophonium, Glycerinester von polymerisiertem Kolophonium, Terpenharze, Cumaron/Inden-Harze, hydrierte Petroleumharze, mit Maleinsäureanhydrid modifiziertes Kolophonium und Kolophoniumderivate, $C_5$-Petroleumharze und Halbester von Styrol/Maleinsäure-Co-polymeren einzeln oder im Gemisch miteinander verstanden. Besonders bevorzugt werden Polyterpenharze aus Alpha- bzw. Beta-Pinen oder modifizierte Glycerinester des Kolophoniums. Diese Harze können je nach den erforderlichen Eigenschaften hinsichtlich der Klebrigkeit und der Haftfestigkeit auf dem Teil, an dem das resultierende Pflaster angebracht werden soll, entweder allein oder in Kombination miteinander verwendet werden.

Antiphlogistika im Sinne der vorliegenden Erfindung sind eines oder mehrere Antiphlogistika der allgemeinen Formel I und/oder II.

Antiphlogistika der allgemeinen Formel I besitzen die folgende Struktur:

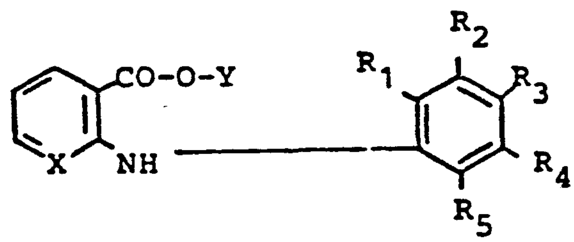

I

wobei

$R_1$ - $R_5$ gleich oder verschieden sein können, und Wasserstoff, Halogen, niederes Alkyl, substituiertes Alkyl,

X N oder CH und

Y Wasserstoff, Metallionen, Alkyl oder substituiertes Alkyl

bedeutet.

Halogen bedeutet Fluor, Chlor, Brom, vorzugsweise Chlor und/oder Brom, besonders bevorzugt Chlor. Niederes Alky ist bevorzugt Alkyl mit 1 - 6 C-Atomen, besonders bevorzugt 1 - 4 C-Atomen, substituiertes Alkyl für $R_1$ - $R_5$ bedeutet bevorzugt Trihalogenalkyl, besonders bevorzugt Trifluoromethyl. Unter Metallionen werden die Ionen der Alkalimetalle, Erdalkalimetalle und des Aluminiums verstanden, vorzugsweise Natrium. Substituiertes Alkyl für Y bedeutet bevorzugt Alkoxy, Alkoxyalkyl, Hydroxyalkyl, Hydroxyalkoxyalkyl, Trihalogenalkyl, wobei die Anzahl der C-Atome 1 bis 6 beträgt und die Alkylkette gerade oder verzweigt sein kann.

Vorzugsweise werden Antiphlogistika der allgemeinen Formel I eingesetzt, in welcher

$R_3$ und $R_4$ Wasserstoff

X Stickstoff oder eine CH-Gruppe,

Y Wasserstoff, $C_1$-$C_4$-Alkyl oder substituiertes $C_1$-$C_4$-Alkyl Hydroxyalkyl oder Hydroxyalkoxyalkyl mit 1 bis 6 C-Atomen,

$R_1$, $R_2$ und $R_5$ Wasserstoff, Chlor, $C_1$-$C_4$-Alkyl oder Trifluormethyl bedeuten.

Besonders bevorzugt sind Antiphlogistika der allgemeinen Formel I, in welcher

X für eine CH-Gruppe steht und

Y Wasserstoff oder Hydroxyalkoxyalkyl mit 1 bis 6 C-Atomen,

$R_1$, $R_2$ und $R_5$ Methyl, Wasserstoff, Trifluormethyl oder Chlor bedeuten.

Ganz besonders bevorzugt sind die folgenden Antiphlogistika:

N-(α, α, α-Trifluor-m-tolyl)-anthranilsäure = Flufenaminsäure

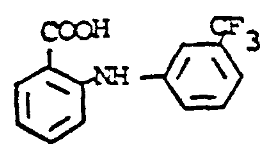

N-(2,3-Xylyl)-anthranilsäure

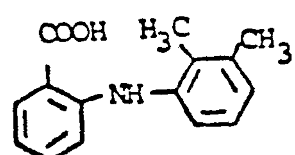

2-(2,6-Xylidino)-nicotinsäure

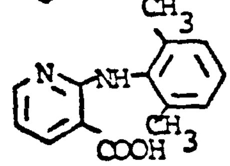

2-(2-Hydroxyethoxy)-ethyl-N-(α,α,α-trifluor-m-tolyl)-anthranilat = Etofenamat

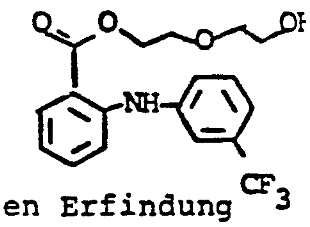

Antiphlogistika im Sinne der vorliegenden Erfindung sind ferner Antiphlogistika der allgemeinen Formel II mit der Struktur:

$$Ar-(-\overset{O}{\overset{\|}{C}}-)_p-(\overset{R}{\overset{|}{C}H})_n-(CH_2)_m-COOH, \qquad II$$

in welcher

R Wasserstoff, niederes Alkyl, substituiertes Alkyl,

Ar Aryl, Heteroaryl, substituiertes Aryl, substituiertes Heteroaryl und

n + m eine ganze Zahl bedeuten und den Wert Null, 1 oder 2 besitzen,

p Null oder 1 bedeutet,

mit der Bedingung, daß Ar nicht Aryl oder Heteroaryl bedeuten, wenn n und m und p den Wert Null besitzen,

sowie deren Ester oder Amide.

Vorzugsweise bedeutet R für niederes Alkyl Reste mit 1 - 6 C-Atomen, bevorzugt 1 - 4 C-Atomen, substituiertes Alkyl, Alkoxyalkyl oder Trihalogenalkyl; Aryl bzw. Heteroaryl, Phenyl, Naphthyl, Thiophenyl, Pyrrolyl, Indenyl, Indolyl, Benzthiazinyl, Phenothiazinyl.

Substituenten für Aryl bzw. Heteroaryl sind Alkyl, bevorzugt grad- und verzweigtkettiges Alkyl mit bis zu 6 C-Atomen, Alkoxy, Oxalkyl, Acyl, Hydroxyl, Acetoxy, Benzoyl, substituiertes Benzoyl, Phenyl, substituiertes Phenyl, Phenoxy, Halogen, Phenylalkenyl, Phenylalkyl.

Die Ester sind Alkylester mit 1 - 6 C-Atomen, bevorzugt 1 - 4 C-Atomen in der Alkoholkomponente, besonders bevorzugt Methyl, Ethyl, i- und n-Propyl, substituiertes Alkyl, z.B. ß-Hydroxyethyl, Ester mit Glycolsäure. Die Amide können in der Gruppierung $-CO-NH_2$ an Stelle eines oder beider Amid-Wasserstoffe auch niedere Alkyle bzw. substituierte Alkyle enthalten.

Besonders bevorzugt sind die folgenden Antiphlogistika der allgemeinen Formel II:

2-Hydroxybenzoesäure

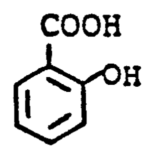

2-Acetoxybenzoesäure

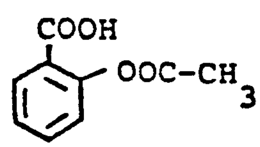

2',4'-Difluor-4-hydroxy-3-biphenylcarbonsäure

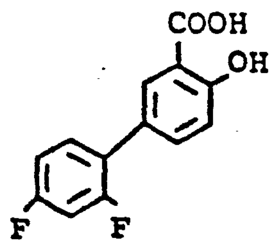

2-Hydroxybenzamid

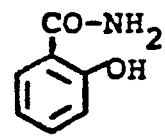

/2-(Aminocarbonyl)phenoxy7
essigsäure

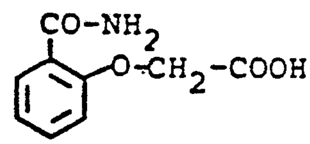

4-Allyloxy-3-chlorphenyl-
essigsäure
=Alclofenac

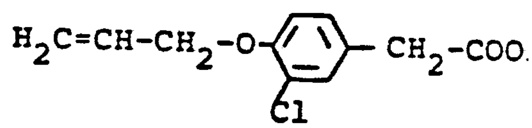

2-/(2,6-Dichlorphenyl)amino7-
phenylessigsäure

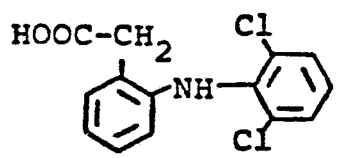

10-Methyl-phenothiazin-2-yl-
essigsäure
= Metiazinsäure

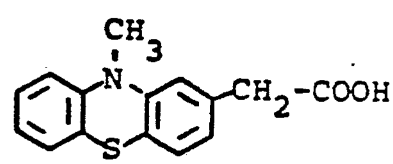

1-Methyl-5-(p-toluoyl)-pyrrol
2-yl-essigsäure

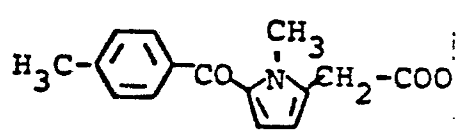

D-2-(6-Methoxy-2-naphthyl)-
propionsäure
= Naproxen

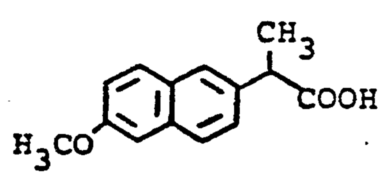

2-(p-Isobutylphenyl)-propionsäure

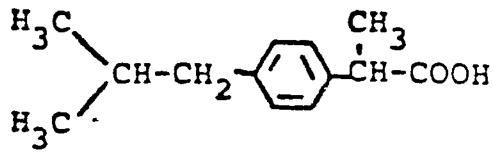

2-(3-Phenoxyphenyl)-propionsäure

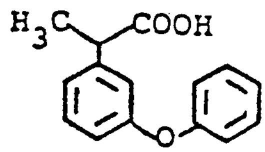

2-(m-Benzoylphenyl)-propionsäure
= Ketoprofen

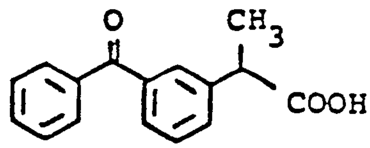

2-[4-(1-Oxo-2-isoindolinyl)-phenyl]-propionsäure
= Indoprofen

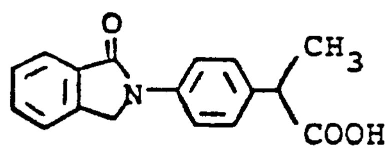

2-(2-Fluorbiphenyl-4-yl)-propionsäure

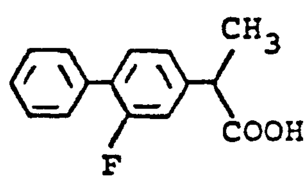

3-(4-Biphenylcarbonyl)-propionsäure

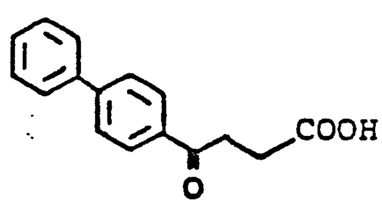

2-(5-Benzoyl-2-thienyl)-
propionsäure

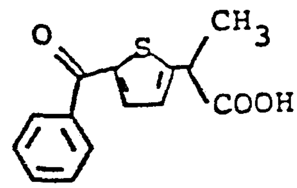

1-(p-Chlorbenzoyl)-5-methoxy
2-methylindol-3-essigsäure

= Indometacin

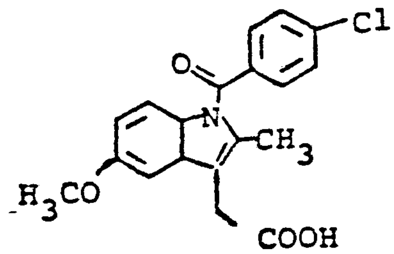

1-(p-Chlorbenzoyl)-5-methoxy-2-
methylindol-3-acetoxyessigsäure

= Acemetacin

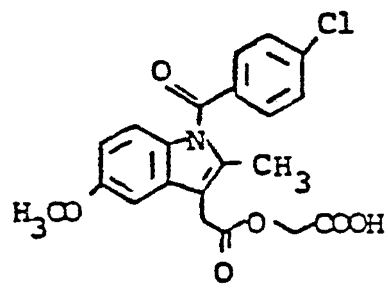

(Z)-5-Fluor-2-methyl-1-([4-methyl-
sulfinyl)phenyl]-methylen)-1H-inden-
3-essigsäure

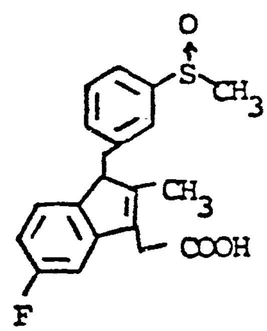

4-Butyl-1,2-diphenyl-3,5-
pyrazolidin-dion

= Phenylbutazon

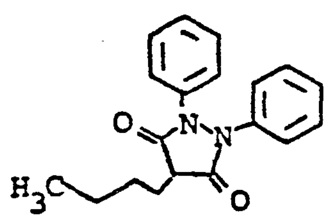

4-(3-Methyl-but-2-enyl)-
1,2-diphenyl-pyrazolidin-
3,5-dion

= Feprazon

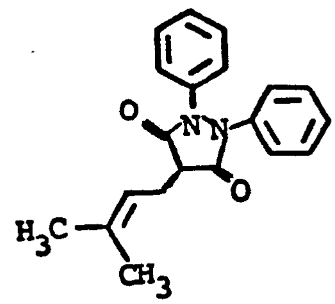

2-(4-Chlorphenyl)-α-methyl-5-
benzoxazol-essigsäure

= Benoxaprofen

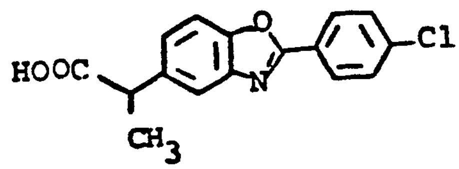

4-Hydroxy-2-methyl-N-2-thiazolyl-
2H-1,2-benzothiazin-3-carboxamid-
1,1-dioxid

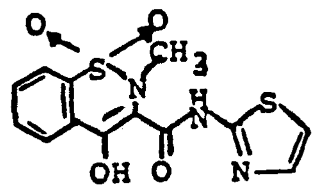

4-Hydroxy-2-methyl-N-2-pyridinyl-
2H-1,2-benzothiazin-3-carboxamid-
1,1-dioxid

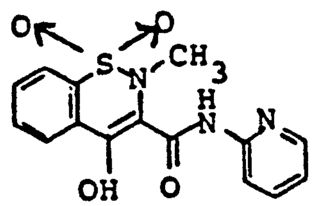

sowie deren Alkylester und substituierte Alkylester.

Die vorstehend genannten Antiphlogistika der allgemeinen Formel I und II können sowohl einzeln als auch zu mehreren in die Pflaster eingearbeitet werden.

Die Antiphlogistika können in einer Menge von 1 - 30 Gew.-%, vorzugsweise 2 - 20 Gew.-% in die Reservoirschicht eingearbeitet werden. Die angegebenen Gew.-% beziehen sich auf Gesamtreservoir.

Diesen Antiphlogistika können zusätzlich noch Wirksubstanzen zugesetzt werden oder auch kühlende oder duftabgebende Substanzen, vorzugsweise Methylsalicylat, Glycolsalicylat, Salicylsäure, Menthol, Pfefferminzöl, Kampfer, Thymol, Acrinol, Scopoliaextrakt, Chlorpheniraminmaleat, Benzylnicotinat, Capsicumextrakt, Nonylvanillylamid, Capsaicin.

Erforderlichenfalls können die erfindungsgemäßen Pflaster mit Additiven und Füllstoffen, z.B. Alterungsschutzmitteln, Antioxidantien und Verstärkungsfüllstoffen versetzt werden, soweit die gelartigen Eigenschaften nicht zerstört werden.

Bekannte Wirkstoffabgabesysteme, wie z.B. Gele, Salbengrundlagen, Pflaster geben ca. 0,5 - 20 % Wirkstoff in 7 Stunden frei. Die oben beschriebenen erfindungsgemäßen Pflaster dagegen setzen in 7 Stunden bis zu 70 % Wirkstoff frei mit einer signifikant größeren Bioverfügbarkeit (3 - 5 mal größer). Die erfindungsgemäßen Systeme können durch Änderung des Polymeranteils, des Schleppmittels bzw. des Harzes bezüglich ihrer Wirkstoffabgabegeschwindigkeit nahezu beliebig eingestellt werden.
Als Deckschicht für die erfindungsgemäßen Pflaster wurden längs- und querelastische Gewirke und Gestricke eingesetzt (s. z. B. Koch-Satlow, Großes Textillexikon, Deutsche Verlagsanstalt Stuttgart 1965).
Gewirke und Gestricke sind demnach textile Flächengebilde, die aus einem oder mehreren Fadensystemen durch Maschenbildung

0154907

auf Wirk- oder Strickmaschinen hergestellt werden. Man unterscheidet zwei Kategorien: Kuliergewirke und -gestricke (Hauptkennzeichen: Fadenverlauf in Querrichtung, analog Schußrichtung bei Geweben) sowie Kettengewirke (Hauptkennzeichen: Fadenverlauf in Längsrichtung, analog Kettrichtung bei Geweben).

Die in der Fachterminologie übliche Begriffstrennung in Gewirke und Gestricke bezieht sich auf den Herstellvorgang. Beim Wirken werden die Maschen einer Maschenreihe gleichzeitig ausgebildet (abgeschlagen), während beim Stricken eine Masche nach der anderen entsteht. Bei der Begriffszuordnung gibt es jedoch Ausnahmen. Bindungstechnisch unterscheiden sich Kulier-Gewirke und -gestricke nicht.

Gewirke und Gestricke besitzen im Gegensatz zu Geweben hohe Dehnung und Elastizität, besonders in Breitenrichtung; des weiteren haben sie infolge der Maschenstruktur ein großes Porenvolumen, was Luftdurchlässigkeit und Thermoisolation begünstigt. Diese und andere Eigenschaften können durch Bindung wie auch Faserstoff- und Garnauswahl weitgehend variiert werden.

Bevorzugt weisen die erfindungsgemäßen verwendeten Gewirke und Gestricke Stretchcharakter auf. Zur Erzielung dieses Stretchcharakters werden die in der Textiltechnik üblichen Methoden eingesetzt (s. Koch-Satlow, Seite 441) oder aber es werden gleich bei der Auswahl der Grundmaterialien für die Gewirke und Gestricke Elastomerfasern bzw. Elastomergarne verwendet.

Neben den Gewirken und Gestricken lassen sich als Deckschicht für die erfindungsgemäßen Pflaster allgemein textile Flächengebilde mit Stretchcharakter verwenden, d. h. alle

dreidimensionalen Gebilde aus natürlichen und synthetischen Textilfasern wie Geflechte, Vliesstoffe oder Filze sind als Deckschicht geeignet.
Als Basismaterial für die Deckschicht kommen u. a. Fasern und Filamente aus Polyamid, Polyester, Polyurethan, Polyamid-Polyurethan, Baumwolle, Zellwolle und tierische Wolle zur Anwendung.

Die textile Deckschicht der erfindungsgemäßen Pflaster wird imprägniert oder beschichtet. Zum Beschichten und Imprägnieren werden die üblichen Techniken und Materialien benutzt (s. auch Koch-Satlow, S. 157 - 159 sowie S. 616 ff.).

Bevorzugt wird die Deckschicht mit Polyisobutylen imprägniert oder beschichtet. Dabei ist die Molmasse des Polyisobutylens bevorzugt > 1.000.000 g/Mol (Viskositätsmittel).

Als Beschichtungs- und Imprägnierungsmaterial kommen bevorzugt die Polyisobutylene in Frage, die auch in der Reservoirschicht enthalten sind, jedoch höhere Molekulargewichte und keine Klebrigkeit aufweisen.

Die Abziehfolie der erfindungsgemäßen Pflaster kann aus okklusiven, flexiblen oder nichtflexiblen Materialien bestehen, wie Polyethylen, Polypropylen, Polyethylenterephthalat, Nylon u. ä. bekannte Folien. Als Abziehfolie können auch Metallfolien, wie Aluminiumfolie, allein oder mit Polymeren laminiert, angewandt werden. Auch mehrschichtige Folien wie Laminate aus Polyethylen mit Polyester-PE-terephthalat und mit Aluminium bedampft, können eingesetzt werden. Andere abziehbare Folien sind u. a. mit Silikon behandelte Polyester, Polyethylenterephthalat mit endständigen Silikongruppen, behandeltes Papier, mit Silikon behandeltes Papier, mit Polyethylen beschichtetes Papier u. ä.

Ein weiterer Gegenstand der Erfindung ist ein Verfahren zur Herstellung medizinischer Pflaster, umfassend eine Deckschicht, eine Wirkstoffreservoirschicht und eine abziehbare Schutzschicht, die im wesentlichen für die Wirkstoffe undurchlässig ist, das dadurch gekennzeichnet ist, daß man eine Polyisobutylen enthaltende Polymerkomponente, ein oder mehrere Schleppmittel und ein oder mehrere Harze in einem Lösungsmittel löst und 1 bis 30 Gew.% Wirkstoff ebenfalls in einem Lösungsmittel zur Lösung bringt, diese Lösungen vereinigt, die vereinigten Lösungen gleichmäßig auf eine für die Wirkstoffe im wesentlichen undurchlässige Deckschicht, die aus einem längs-querelastischen, mit einem Polymer imprägnierten oder beschichteten textilen Flächengebilde, insbesondere Gewirk oder Gestrick besteht, aufträgt und trocknet und gegebenenfalls die getrocknete Deckschicht auf der beschichteten Seite mit einer abziehbaren Schutzschicht versieht.

0154907

## Herstellungsbeispiele

### Beispiel 1

Eine 12,5 %ige Polyisobutylenlösung (M. G. Viskositätsmittel 1.270.000) (in Benzin), wird auf silikonisiertem Papier aufgetragen und einem Gewirk bestehend aus Polyamid-Polyurethanfasern zukaschiert und im Trockenkanal stufenweise bei 70 / 90 / 110 °C getrocknet (Polymer 30 $g/m^2$).

Auf silikonisiertem Papier wird eine in Benzin/Aceton gelöste Mischung bestehend aus
36,000 g Polyisobutylen M.G. Viskositätsmittel 400.000,
44,928 g Paraffin dünnflüssig,
9,000 g Polyterpenharz aus ß-Pinen,
10,000 g Etofenamat und
0,072 g Alterungsschutzmittel
aufgetragen und im Trockenkanal stufenweise bei 70 / 90 / 100 °C getrocknet (Wirkstoffabgabesystem ca. 150 $g/m^2$).

Nach dem Trocknen wurde das mit Polyisobutylen imprägnierte Gewirk mit Stretchcharakter zukaschiert.

### Beispiel 2

Auf silikonisiertem Papier wurde eine Polymerlösung (Benzin/Aceton) bestehend aus
36,000 g Polyisobutylen M.G. Viskositätsmittel 1.270.000,
44,928 g Paraffin dünnflüssig,
9,000 g Polyterpenharz aus α-Pinen,
10,000 g Etofenamat und
0,072 g Alterungsschutzmittel aufgetragen und im Trockenkanal stufenweise bei 70 / 90 / 110 °C getrocknet.

0154907

Nach dem Trocknen wurde das Wirkstoffabgabesystem mit Polyisobutylen (wie in Beispiel 1) auf beschichtetes Stretchmaterial kaschiert.

Die erfindungsgemäßen Pflaster besitzen eine ebenso gute Resorption der Wirkstoffe wie herkömmliche mit Aluminium-Polyethylen-Folien beschichtete Pflaster.

Zum Kaschieren des Wirkstoffdepots auf die textilen Flächengebilde mit Stretchcharakter werden u. a. die Methoden und Materialien eingesetzt, die in Koch-Satlow, Großes Textillexikon, beschrieben werden.

Patentansprüche

1. Medizinische Pflaster,umfassend eine Deckschicht, eine Wirkstoffreservoirschicht und eine abziehbare Schutzschicht, die im wesentlichen für die Wirkstoffe undurchlässig ist, dadurch gekennzeichnet, daß die Reservoirschicht 1 bis 30 % Wirkstoff in einem Elastomergemisch, bestehend aus Polyisobutylen, Polybutadienöl und/oder Paraffinöl und klebrigmachenden Harzen, enthält und die Deckschicht im wesentlichen für die Wirkstoffe undurchlässig ist und aus einem längs-querelastischen mit einem Polymer imprägnierten oder beschichteten textilen Flächengebilde besteht.

2. Medizinische Pflaster zur Verabreichung von Wirkstoffen durch die Haut, enthaltend eine Deckschicht, eine wirkstoffhaltige Reservoirschicht, welche ein Polymer, ein Schleppmittel, ein Harz und eine abziehbare Schutzschicht enthält, dadurch gekennzeichnet, daß die Polymeren der wirkstoffhaltigen Reservoirschicht Polyisobutylene und/oder Copolymere des Isobutylens mit 1 bis 5 Mol% konjugierten Dienen sind, die eine Molmassenverteilung $M_w/M_n$ von 1,5 bis 3,5, vorzugsweise von 2,0 bis 3,0 und ein Viskositätsmittel der Molmasse von 30.000 bis 4.000.000 aufweisen und als Wirkstoffe 2 bis 30 Gew.-% Antiphlogistika im Reservoir enthalten sind und die Deckschicht im wesentlichen aus einem für die Wirkstoffe undurchlässigen, längs-querelastischen, mit einem Polymer imprägnierten oder beschichteten textilen Flächengebilde besteht.

3. Medizinische Pflaster nach Anspruch 1, dadurch gekennzeichnet, daß das wirkstoffhaltige Reservoir neben den Wirkstoffen 30 bis 60 Gew.% Polymer, 30 bis 60 Gew.% Schleppmittel und 2 bis 40 Gew.% Harz enthält.

4. Medizinische Pflaster nach Anspruch 1 und 2, dadurch gekennzeichnet, daß als Polymer im Reservoir Polyisobutylene mit Viskositätsmitteln der Molmasse von 50.000 bis 1.000.000 g/Mol, vorzugsweise von 80.000 bis 500.000 g/Mol eingesetzt werden.

5. Medizinische Pflaster nach Anspruch 1 und 2, dadurch gekennzeichnet, daß als Polymer im Reservoir Copolymere des Isobutylens mit 1 bis 5 Mol% konjugierter Diolefine, bevorzugt mit $C_4$-$C_6$ Dienen, besonders bevorzugt mit Isopren eingesetzt werden.

6. Medizinische Pflaster nach Anspruch 1 und 2, dadurch gekennzeichnet, daß sie in oder auf der Deckschicht Polyisobutylen mit Viskositätsmitteln der Molmasse $>$ 1.000.000 g/Mol enthalten.

7. Medizinische Pflaster nach einem oder mehreren der Ansprüche 1 bis 6, dadurch gekennzeichnet, daß als Wirkstoff Antiphlogistika, vorzugsweise solche der allgemeinen Formel I

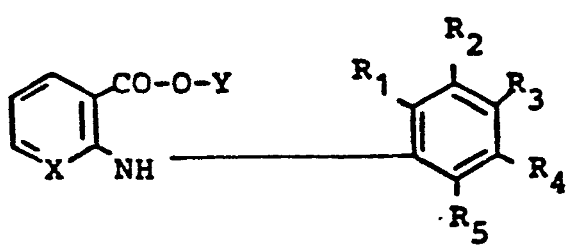

I

wobei

$R_1$ - $R_5$ gleich oder verschieden sein können und Wasserstoff, Halogen, niederes Alkyl, substituiertes Alkyl,

X N oder CH und

Y Wasserstoff, Metallionen, Alkyl oder substituiertes Alkyl

bedeutet,

und/oder der Formel II,

$$Ar-(-\overset{O}{\overset{\|}{C}}-)_p-(\overset{R}{\overset{|}{C}}H)_n-(CH_2)_m-COOH, \qquad II$$

in welcher

R Wasserstoff, niederes Alkyl, substituiertes Alkyl,

Ar Aryl, Heteroaryl, substituiertes Aryl, substituiertes Heteroaryl und

n + m eine ganze Zahl bedeuten und den Wert Null, 1 oder 2 besitzen,

p Null oder 1 bedeutet,

mit der Bedingung, daß Ar nicht Aryl oder Heteroaryl bedeutet, wenn n und m und p den Wert Null besitzen,

eingesetzt werden.

8. Medizinische Pflaster nach einem oder mehreren der Ansprüche 1 bis 7, dadurch gekennzeichnet, daß sie als Wirkstoff Etofenamat enthalten.

9. Medizinische Pflaster nach einem oder mehreren der Ansprüche 1 bis 7, enthaltend Etofenamat und/oder Ketoprofen.

10. Verfahren zur Herstellung medizinischer Pflaster, umfassend eine Deckschicht, eine Wirkstoffreservoirschicht und eine abziehbare Schutzschicht, die im wesentlichen für die Wirkstoffe undurchlässig ist, dadurch gekennzeichnet, daß man eine Polyisobutylen enthaltende Polymerkomponente, ein oder mehrere Schleppmittel und ein oder mehrere Harze in einem Lösungsmittel löst und 1 bis 30 Gew.-% Wirkstoff ebenfalls in einem Lösungsmittel zur Lösung bringt, diese Lösungen vereinigt, die vereinigten Lösungen gleichmäßig auf eine für die Wirkstoffe im wesentlichen undurchlässige Deckschicht, die aus einem längsquerelastischen, mit einem Polymer imprägnierten oder beschichteten textilen Flächengebilde, insbesondere Gewirk oder Gestrick besteht, aufträgt und trocknet und gegebenenfalls die getrocknete Deckschicht auf der beschichteten Seite mit einer abziehbaren Schutzschicht versieht.